# EUROPEAN PATENT SPECIFICATION

(11) **EP 1 219 314 B1**
(45) Date of publication and mention of the grant of the patent: **17.03.2004**
(21) Application number: 00311778.5
(22) Date of filing: 29.12.2000
(51) Int. Cl.: A61M 11/00, A61M 15/00, A61M 16/20, A61M 16/18

(54) **Liquid discharge apparatus having magnetic valve**
Flüssigkeitsausstossvorrichtung mit Magnetisch betätigbares Ventil
Dispositif à projection de liquide avec soupape électromagnétique

(43) Date of publication of application: 03.07.2002
(73) Proprietor: INSTRUMENTARIUM CORPORATION, 00510 Helsinki (FI)
(72) Inventor: Haveri, Heikki, 00360 Helsinki (FI)
(74) Representative: Charlton, Peter John

(56) References cited:
- EP-A- 0 794 370
- WO-A-99/22796
- US-A- 4 210 174
- US-A- 4 630 799
- US-A- 5 388 571
- US-A- 5 443 059

## Description

### BACKGROUND OF THE INVENTION

The present invention relates to an improved apparatus, such as a nebulizer apparatus for discharging liquids. Nebulizers, or atomizers, are devices that generate a fine spray or aerosol, usually of liquid. A particularly useful application for nebulizers is to provide a fine spray containing a dissolved, or a suspended particulate or colloidal, pharmaceutical agent for administration to a subject by inhalation. Such inhalation treatment is highly effective for conditions affecting the subject's respiratory organs. Further, since the lungs are close to the heart and the blood circulatory system of the body, drug administration by inhalation provides an effective and rapid delivery system to all organs of the body. Other applications for liquid discharge apparatus include dispensing insecticides, paint or other coating, deodorants, water for humidification, etc. Other devices that may incorporate the present invention include printers in which ink is discharged onto paper.

When dispensing a pharmaceutical agent, the nebulizer may be placed directly in the mouth or nose of the subject so that the spray can be entrained in the respiratory gases inhaled during normal, spontaneous breathing of the subject In other cases, the subject breathes with the aid of a respiratory ventilator. A typical ventilator has a breathing circuit comprising an inhalation limb and an exhalation limb connected to two arms of an Y-connector. The third arm of the Y-connector is connected, via a patient limb, to a mouthpiece, mask, or endotracheal tube for the subject The ventilator provides a complete or partial supply of breathing gas to the subject through the inhalation limb during inhalation. The contraction of the subject's lungs discharges gases through the exhalation limb during exhalation. When a nebulizer is employed in conjunction with a ventilator, it is typically placed in the patient limb but can also be placed in the inhalation limb.

Nebulizers currently in use for ventilator applications generate the spray either pneumatically or by means of ultrasonic vibrations. Pneumatic nebulizers are typically used with a liquid, such as an aqueous drug solution. High pressure driving gas is conducted through a nozzle to draw the drug from a drug supply for the nebulizer. The drug is discharged against a baffle or other means in a gas space of the nebulizer, breaking the liquid into a fine spray. The gas space is in fluid communication with the inhaled gas pathway of the breathing circuit so that the gas flow discharged from the nozzle along with the nebulized drug is conducted to the breathing circuit and ultimately to the subject.

Disadvantages in the use of pneumatic nebulizers include the following. If the nebulizer adds a significant quantity of gas, for example, up to five liters/minute, into the breathing circuit, the breathing gas composition may be affected. Due to passage of the driving gas through the nozzle, impingement of the drug on the baffle, etc., pneumatic nebulizers are noisy. And, controlling the commencing and stopping of the drug agent spray is difficult and is not very accurate, resulting in wastage of the drug.

The foregoing shortcomings of pneumatic nebulizers have led to the use of ultrasonic nebulizers in which the fine spray is produced by ultrasonic vibration of the liquid, as by a piezoelectric crystal. Breathing gas composition and the on-off operation are easier to control with such nebulizers than with a pneumatic nebulizer. However, existing ultrasonic devices may require a large, bulky electrical power supply to power the crystal and may not be able to nebulize colloidal or particulate suspensions.

Tn one type of ultrasonic nebulizer, the fine spray is produced by dropping the liquid on, or otherwise applying it to, the vibrating element. See U.S. Patent 5,443,059. U.S. Patent 3,812,854 describes another type of nebulizer for inhalation therapy in which the spray is generated on the front surface of a vibrating, porous body. The pores of the body form a network of passages that enable the liquid to flow through the body. The liquid to be nebulized is supplied under pressure from a liquid supply through a liquid conduit to the pores, and forced through the pores to the front surface of the porous body where it is discharged as a spray. U.S. Patent 5,487,378 describes a nebulizer in which the aerosol is formed using a mesh plate instead of a porous solid body. The mesh plate has a plurality of orifices for the liquid. The liquid or the nozzle assembly is vibrated ultrasonically by a piezoelectric element to nebulize a dose of liquid as it passes through the mesh plate.

U.S. Patents 5, 518,179 and 5,299,739 describe nebulizers in which capillary feed is used to supply liquid to the vibrating clement. A further alternative for liquid supply is achieved by condensing a liquid vapor on one face of a membrane, the liquid thus condensed being dispensed in droplet form. See U.S. Patent 5,518,179.

U.S. Patent 5,938, 117 describes an apparatus for dispensing liquids as an atomized spray having a fluid supply system that transports liquid to an apertured oscillating surface. The fluid supply system is connected to an electronic flow control valve. The valve is connected to an electronic circuit. In the event of the delivery of excess liquid, the surface oscillation amplitude decreases and the current draw by the piezoelectric element decreases. A current sensor circuit senses the current draw and transmits an overflow signal to the flow control valve to reduce the delivery rate of liquid to the surface until the amount of fluid returns to a normal level.

A specific difficulty with current liquid discharge apparatus, such as nebulizers, is the clinical aspect. Some parts of the nebulizer may come into contact with the breathing gas of the patient and those parts should be carefully cleaned or changed when changing between subjects to minimize bacteria infections. Other parts of the nebulizer may, furthermore, be in contact with different anaesthetic agents in the breathing gases, which are specific to each subject. Those parts should also be cleaned or changed when changing to a different patient. Still other parts of the nebulizer are in contact with the pharmaceutical drug administered to a given subject. These parts should be cleaned when changing to a different drug or a different subject.

The foregoing problems have increased the cost and complexity, and reduced the ease of use of liquid discharge apparatus.

### BRIEF SUMMARY OF THE INVENTION

An object of the present invention is to provide an improved apparatus having an accurate liquid supply system to supply liquid to a displaceable element so that it can be efficiently discharged from the apparatus. Another object of the invention is to provide an apparatus having a liquid supply system in which those parts that are exposed to the liquid during operation are changeable or/and disposable to reduce the cleaning work when changing liquids or, if the apparatus is a drug dispensing nebulizer, when changing subjects.

The above and other objects are attained by a liquid discharge apparatus comprising liquid discharge means, such as a mesh plate connected to a piezoelectric vibrating means. The apparatus also comprises a liquid supply system including a valve having, in one embodiment, a fixed part and a changeable or disposable part to control the liquid flow to the liquid discharge means via a liquid conveying means. The liquid supply system has a liquid reservoir attached to the changeable part of the valve from which the liquid is fed to the valve. The fixed part of the valve includes an electromagnet. A ferromagnetic member is positioned in the magnetic field to control liquid flow through the liquid conveying means. A liquid detection system detects the liquid arriving at the liquid discharge means from the liquid reservoir and controls the valve which regulates the liquid flow using the fixed part of the valve.

In another embodiment, the valve is placed in the liquid reservoir and the liquid supply system includes an actuator for the valve.

Various other features, objects, and advantages of the invention will be made apparent from the following detailed description and the accompanying drawing.

### BRIEF DESCRIPTION OF THE SEVERAL VIEWS OF THE DRAWING

In the drawings:
Fig. 1 is a general cross sectional view of the liquid discharge apparatus of the present invention, the operating environment for the apparatus being shown in generalized schematic form;
Fig. 2 is an exploded, cross sectional view showing the apparatus of the present invention;
Figs. 3a and 3b are cross sectional views showing the filling of the liquid reservoir of the apparatus of Fig. 1;
Figs. 4a and 4b are schematic views showing the operation of the vibrating and atomizing means of the apparatus of Fig. 1;
Fig. 5a-1 and 5a-2 are cross-sectional schematic views of components of a valve incorporated in the liquid discharge apparatus;

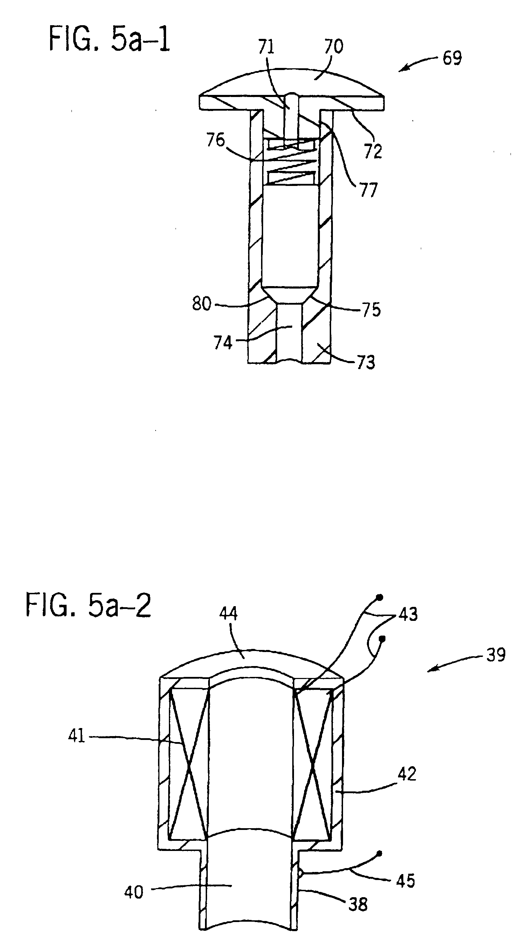

Figs. 5b-1 and 5b-2 are generally orthogonal views of one embodiment of the part of the valve shown in Fig. 5a-1 that controls liquid flow from through the valve;
Figs. 5b-3 and 5b-4 are generally orthogonal views of another embodiment of the part of the valve shown in Fig. 5a-a that controls liquid flow through the valve;

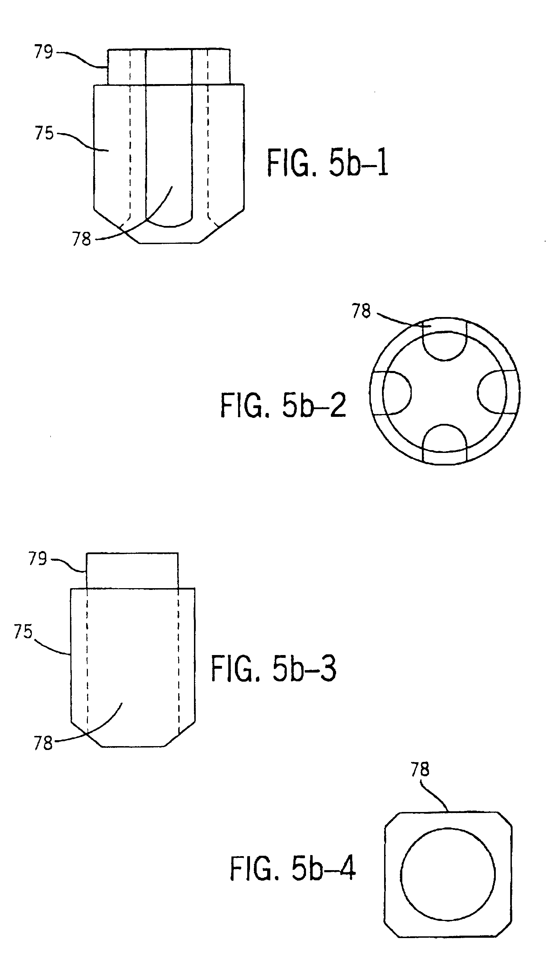

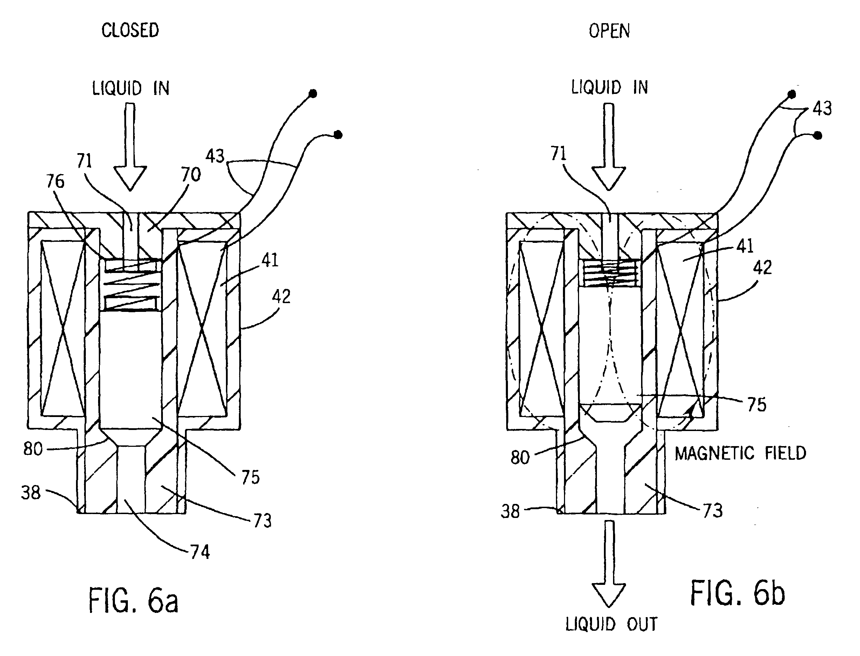

Figs. 6a and 6b are cross-sectional, schematic views showing operation of the valve of Fig. 5;

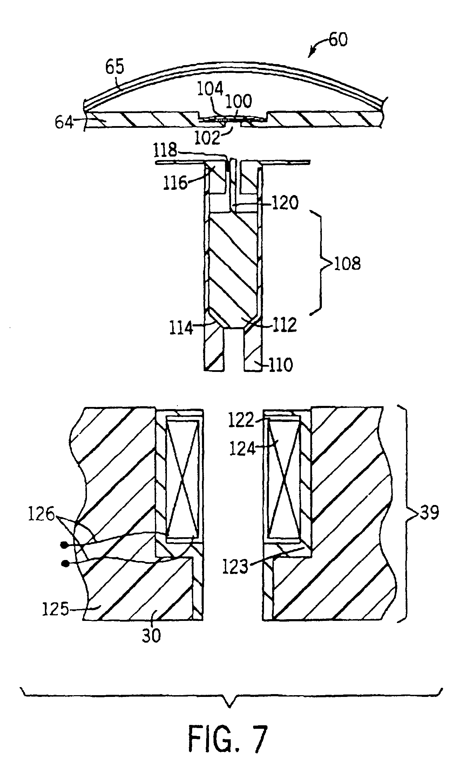

Figs. 7 is a cross sectional, exploded schematic view of another embodiment of a valve suitable for use in the liquid discharge apparatus nf the present invention;

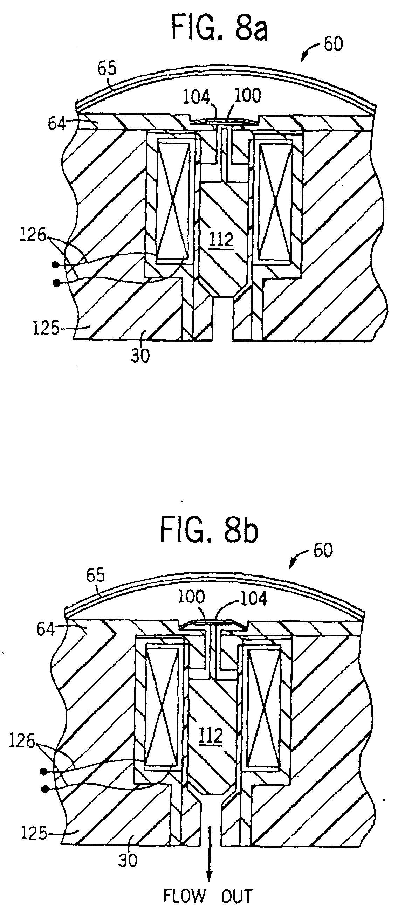

Figs. 8a and 8b are cross sectional, schematic views showing the operation of the valve of Fig. 7;

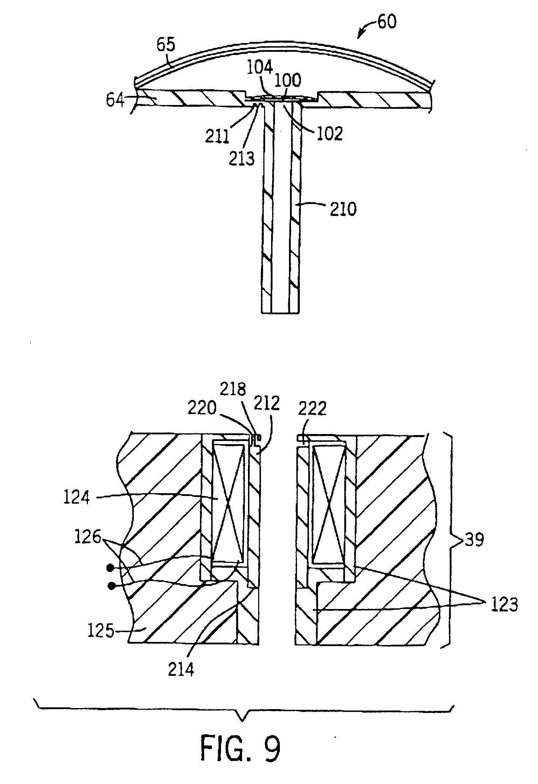

Fig. 9 shows a further embodiment of a valve suitable for use in the liquid discharge apparatus of the present invention; and

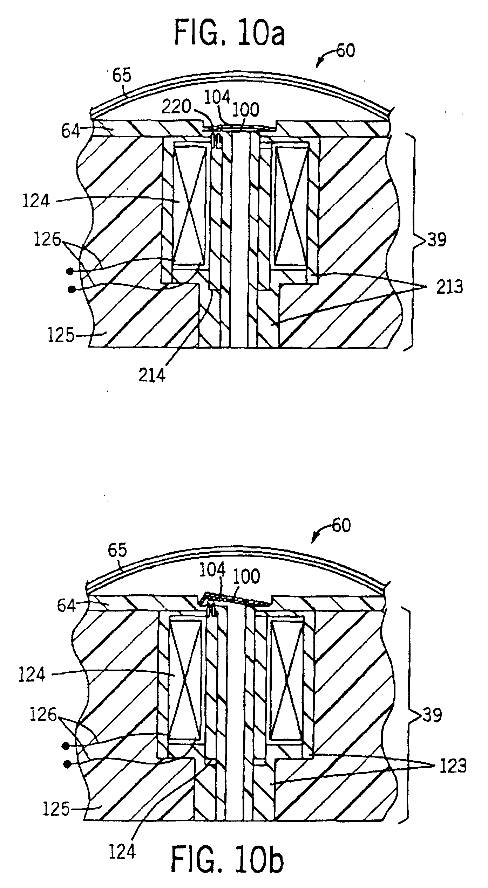

Fig. 10a and 10b are cross sectional schematic views showing the operation of the valve of Fig. 9.

### DETAILED DESCRIPTION OF THE INVENTION

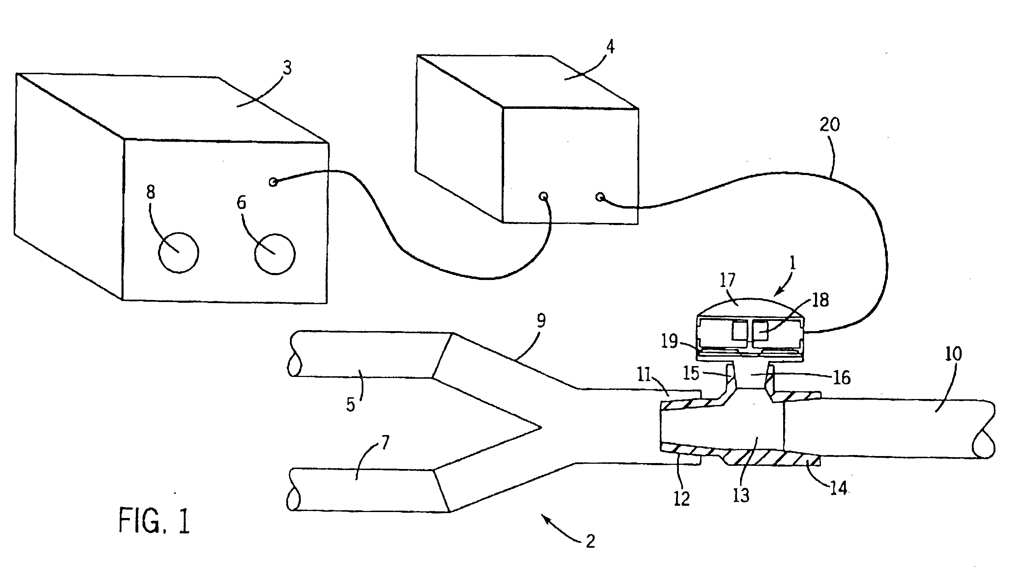

Liquid discharge apparatus 1 of the present invention is shown as a nebulizer in Fig. 1 and is typically used in conjunction with breathing circuit 2, ventilator 3 and control unit 4. The nebulizer apparatus 1 atomizes liquid solutions or suspensions for delivery to a subject, as for example as a drug treatment for a patient. Breathing circuit 2 includes inhalation limb 5, which is coupled to ventilator 3 at inhalation limb connector 6. Exhalation limb 7 is connected to ventilator 3 at exhalation limb connector 8. Inhalation limb 5 and exhalation limb 7 are connected to two arms of Y-connector 9. A third arm of Y-connector 9 is connected to one end of patient limb 10. The other end of patient limb 10 is directed to a mouthpiece, facemask, or endotracheal tube for the subject.

Ventilator 3 provides all or a portion of the breathing gases for the subject by providing inhalation gases in inhalation limb 5. The inhalation gases pass through Y-connector 9 and into patient limb 10 for supply to the subject On exhalation, the breathing gases pass through patient limb 10, Y-connector 9, and exhalation limb 7 back to ventilator 3.

As shown in Fig. 1, the nebulizer apparatus 1 is preferably positioned in patient breathing circuit 2 as near the subject as possible to minimize the aerosol transport path, and to minimize the deposition of the aerosol on the walls of the breathing circuit. To this end, nebulizer apparatus 1 may be inserted in the breathing circuit between Y-connector 9 and patient limb 10. The Y-connector 9 has a socket 11 for receiving tubular projection 12 of adapter 13 for nebulizer apparatus 1. The tubular socket 14 of the adapter 13 receives the patient limb 10. The adapter 13 also has a tubular socket 15 in which nebulizer apparatus 1 is placed. Nebulizer apparatus 1 has tubular projection 16 for this purpose. When nebulizer apparatus 1 is not needed, or when the nebulizer apparatus is removed for cleaning or maintenance, a cap (not shown) may be fitted into or over the opening of socket 15 to allow breathing circuit 2 to function in a normal manner. Alternatively, the entire adapter 13 and nebulizer apparatus 1 may be removed from the breathing circuit, and patient limb 10 reconnected directly to Y-connector 9. Control unit 4 is typically located separately from nebulizer apparatus 1 and may be incorporated in ventilator 3, if desired.

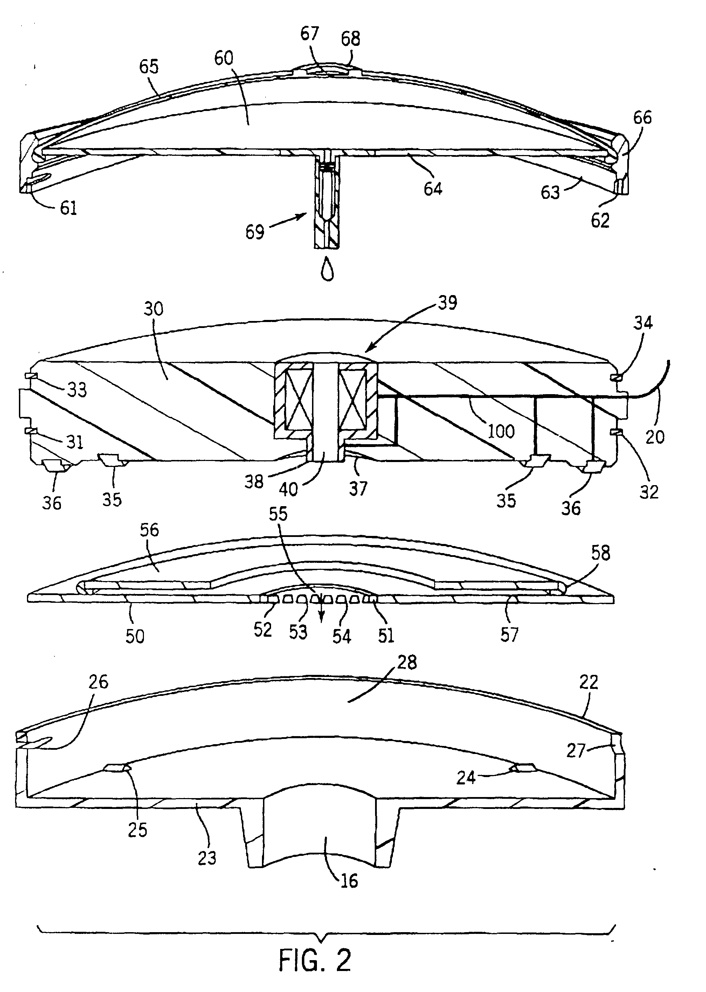

Nebulizer apparatus 1 includes a liquid reservoir 17 for material to be nebulized. In the embodiment shown in the drawings, reservoir 17 is removably mounted on nebulizer apparatus 1. Alternatively it may be remote from nebulizer apparatus 1. A two-part, liquid flow controlling valve 18 is provided between the liquid reservoir 17 and the atomizing means 19 of nebulizer apparatus 1. In the embodiment of the invention shown in Figs. 2-6, one part of the valve is attached to removable reservoir 17. The other part of the valve is placed inside the body of nebulizer apparatus 1. The two parts together form control valve 18. Electrical control signals are supplied to control valve 18 via cable 20 from control unit 4.

As noted above, the material to be nebulized can comprise a solution, or a particulate or colloidal suspension, of a product, such as a pharmaceutical agent. For purposes of explanation, the material undergoing nebulization is hereinafter generally described as a liquid.

Nebulizer apparatus 1 is shown in detail in the cross sectional, exploded view of Fig. 2. Nebulizer apparatus 1 shown in Fig. 2 has an annular housing 22 with the tubular projection 16 in the lower part of it that mounts the apparatus in socket 15 of adapter 13. Housing 22 may be formed of plastic or similar material. The planar base member 23 formed on the lower edge of housing 22 contains circumferentially spaced projecting parts 24 and 25. Housing 22 is attached to internal plug member 30 by a spiral or bayonet fastening formed, in part by openings 26 and 27. Associated projections 31 and 32 are situated symmetrically on opposite sides of plug member 30 and fit into openings 26 and 27 formed in the housing 22. Plug member 30 may be separated from, or joined to, housing 22 by turning and pulling or turning and pushing plug member 30 with respect to housing 22. This allows the portions of apparatus 1 carrying out the nebulizing of the liquid, positioned in cavity 28 of housing 22, to be removed at the end of therapy for replacement, or for cleaning when a different pharmaceutical agent is to be administered to the subject.

Projecting parts 24 and 25 separate the disc-like plate 50 from planar base member 23, preventing the opposing faces of the elements from touching. Plate 50 is I shown with enlarged thickness in Fig. 2. The diameter of plate 50 is smaller than the diameter of cavity 28. Plate 50, made of a conductive material such as brass, contains a central opening 51. A mesh plate 52, having at least one hole 53, is attached to plate 50 in central opening 51. Mesh plate 52 may be mounted to plate 50 by gluing with glue, brazing, welding, or other suitable technique. Plate 50 and mesh plate 52 may be formed from a single sheet of material, if desired.

For a nebulizer, mesh plate 52 is a relatively thin plate having a plurality of holes 53. Mesh plate 52 may be about 0.02 mm thick. The diameter of the holes at front surface 54 is preferably approximately 2-10 µm. Such holes may be formed in the plate by an electroforming process, which process produces holes of increasing diameter toward rear surface 55. However, straight holes will work equally well, the primary criterion being that the exit diameter in front surface 54 of mesh plate 52 is such as to form droplets of the desired size.

Front surface 54 of mesh plate 52 is exposed to the pressure of the breathing gases in breathing circuit 2. These pressures will vary during inhalation and exhalation conditions in breathing circuit 2. For example, with artificial ventilation, breathing circuit pressures may increase up to 100 mbar during inspiration and thereafter decrease during expiration. Projecting parts 24 and 25 separating plate 50 from the planar member 23 form a pressure balancing channel between the plate 50 and planar member 23 together with the spacing between the plate 50 and the inside surface of the side wall of housing 22. These channels connect the volume between plug member 30 and plate 50 with breathing circuit 2 for equalizing the prevailing pressure at both sides of mesh plate 52. The pressure balancing provided by the channels prevents breathing gas from flowing through the holes in mesh plate 52, in opposition to the liquid being nebulized, which flow might degrade the operation of nebulizer apparatus 1. It also avoids pressure stressing mesh plate 52 and causing leaks to occur through the mesh plate.

Means are provided in apparatus 1 to displace plate 50 to discharge liquid. This means may comprise a ring-like vibrating element, such as a piezoelectric element 56, is mounted adjacent the upper surface of plate 50. Specifically, the piezoelectric element 56 is spaced from plate 50 by a small gap 57 and secured to plate 50 about its periphery by conductive glue, brazing, welding, or other suitable technique, shown as 58 in Fig. 2. Piezoelectric element 56 has a central opening coaxial with the central opening 51 of plate 50.

Plug member 30 is formed from a non-conductive material, such as plastic, and is placed in the cavity 28 defined by the housing 22. Lower part of plug member 30 has two power terminals 35 and 36 made of conductive material. Plug member 30 is placed on top of plate 50 so that the plate is between the electrodes and projecting parts 24 and 25. The terminal 35, which terminal may be in the form of a ring- shaped spring, contacts piezoelectric element 56 and is connected to cable 20 through the plug member 30. A second electrical power terminal 36, also connected to cable 20 through plug member 30, contacts conductive plate 50. Terminal 36 may be electrically grounded for purposes of applying a voltage to piezoelectric element 56 in conjunction with terminal 35.

Tubular sensing electrode 38, which is close to the upper surface of mesh plate 52, is used for impedance measurement of the presence of liquid in nebulizer apparatus 1 in conjunction with plate 50 which is opposite the sensing electrode. A small domed cavity 37 may be formed in the lower surface of plug member 30 to surround sensing electrode 38. As hereinafter noted, tubular sensing electrode 38 also forms part of a ferromagnetic body for control valve 18.

Valve 18, which is used for controlling the supply of the liquid to vibrating mesh plate 52, is comprised of two components including a fixed part 39 and a changeable or disposable part 69. The fixed part 39 is located inside plug member 30 and the disposable part 69 is affixed to the liquid reservoir. The disposable part 69 is designed in a tubular form to extend through the cavity 40 of the fixed part 39, when the reservoir is attached to plug member 30. The disposable part 69 is capable, by itself, of functioning as a shut off for the reservoir, but, in conjunction with fixed part 39, it also functions as a valve which is used to regulate the liquid flow from the reservoir to vibrating mesh plate 52.

Reservoir 17, identified more particularly as 60 in Figs. 2 et seq., is attached to the top of plug member 30 by spiral or bayonet fastening formed by openings 61 and 62. Associated projections 33 and 34 are situated symmetrically on the sides of plug member 30 and fit into openings 61 and 62 formed in the reservoir 60. Reservoir 60 may be fastened to, or unfastened from, plug member 30 by turning and pushing or turning and pulling the reservoir with respect to plug member 30. This allows the reservoir to be removed at the end of therapy for replacement, or when a different drug is to be administered to the subject.

Reservoir 60 comprises a peripheral member 63, a plate 64 made of material such as plastic, an elastic membrane 65 made of a material such as rubber, and the changeable or disposable part 69 of valve 18 affixed to the bottom of plate 64. Membrane 65 is stretched on the top of plate 64 and the two elements then pushed into peripheral member 63. Membrane 65, which is between plate 64 and peripheral member 63, is locked in the channel 66 by the plate 64. Reservoir 60 may alternatively be formed as a single part by using the modem multi component fabrication techniques using plastics.

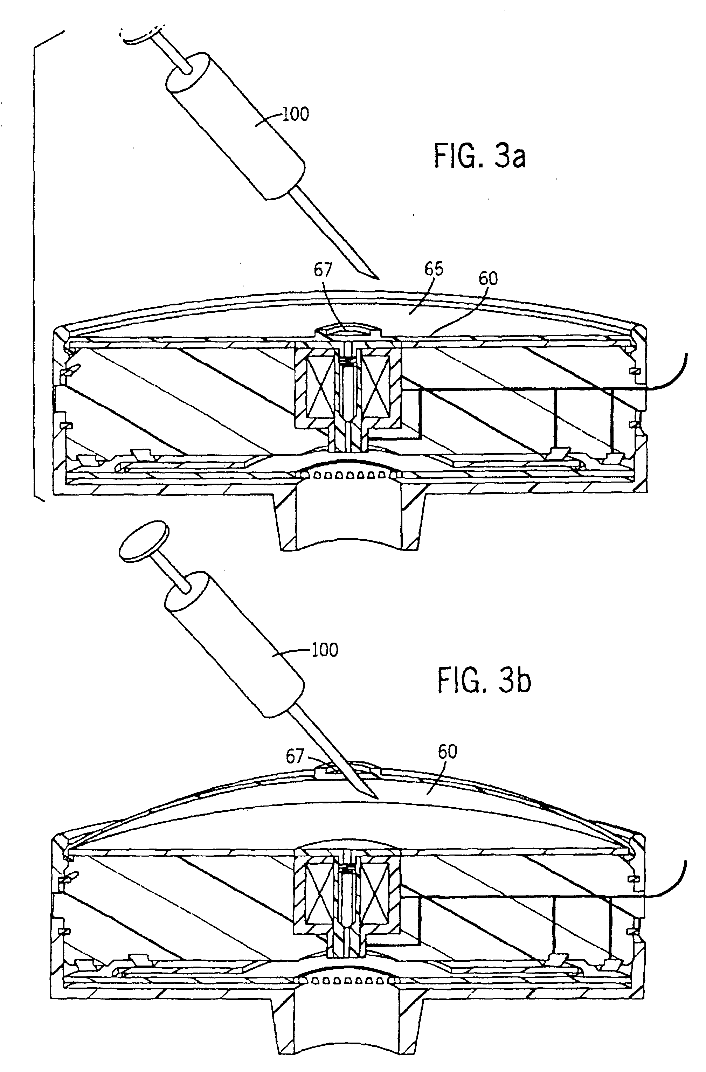

In the middle of membrane 65, on the upper side, is a thicker area 67 of the membrane separated by ring 68. Reservoir 60 is filled with a liquid to be atomized by injecting the liquid with a syringe through area 67 inside the ring 68. Specifically, when the reservoir is empty, membrane 65 is in the pre-strained state shown in Fig. 3a, which establishes enough pressure to empty the space beneath the membrane of liquid inside the reservoir. When the reservoir is filled with liquid by injecting it with a syringe through the membrane in the area 67, as shown in Fig. 3b, pressure inside the reservoir increases as the membrane stretches.

In operation, valve 18, which is used for supplying the liquid to vibrating mesh plate 52, is opened in response to a signal from cable 20 and liquid flows from reservoir 60 toward the mesh plate 52. The liquid flows out through the disposable part 69, which is placed inside the cavity of fixed part 39 and tubular sensing electrode 38, into contact with the upper surface of mesh plate 52. The cohesive forces in the liquid create a column of liquid extending between the lower end of sensing electrode 38 and mesh plate 52. To control the transport of liquid from reservoir 60 to mesh plate 52, the sensing electrode 38 and the mesh plate 52 function as sensing electrodes that detect the presence of liquid between the lower end of sensing electrode 38 and the rear surface 55 of mesh plate 52. The detection is based on alteration of the impedance between the two elements that function as sensing electrodes.

With the continued supply of liquid, the increased column of liquid formed between the end of sensing electrode 38 and the rear surface 55 of mesh plate 52 will significantly alter this impedance. A signal from mesh plate 52 is obtained via terminal 36 and a signal from sensing electrode 38 is obtained via conductor 100. The signals from the two sensing electrodes are inputted to an impedance sensor inside the control unit 4 via cable 20 and are used by the control unit 4 to close valve 18 to terminate or reduce the supply of liquid. When the impedance between the electrodes changes due to the liquid receding away from the end of sensing electrode 38 and the rear surface 55 of mesh plate 52 during operation of nebulizer apparatus 1, the valve opens again to allow the flow of liquid from the reservoir. The delivery of liquid to be nebulized can be controlled either by continuously vibrating mesh plate 52 and continuously regulating the liquid supply or by regulating the activation of mesh plate vibration and intermittently supplying liquid when the amount of liquid between the mesh plate 52 and the end of sensing electrode 38 is reduced.

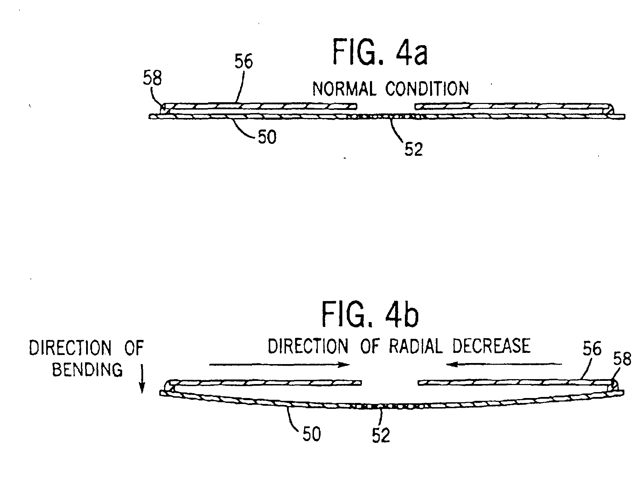

For a nebulizer, high frequency voltage is supplied from a power source inside control unit 4 though cable 20 and terminals 35 and 36 to piezoelectric vibrating element 56 to cause the element to vibrate. The voltage causes the element 56 to contract from the normal condition, shown in Fig. 4a, to a radially decreased condition shown in Fig. 4b and return to the normal condition. Due to the joinder of piezoelectric element 56 to plate 50 about the periphery of the element, the radial size reduction of piezoelectric clement 56 causes plate 50 to deflect or displace as by bowing, as shown in Fig. 4b, and then return to the flat condition, shown in Fig. 4a, when piezoelectric element 56 returns to the normal state. The action of plate 50 shown in Figs. 4a and 4b discharges nebulized liquid from holes 53 in mesh plate 52. At the front surface 54 of the vibrating mesh plate 52, the atomized liquid will grow into drops at each hole 53 due to the liquid surface tension. The drops will increase in size until the expelling forces arising from the movement of mesh plate 52 and the mass of each drop exceeds the holding force determined by the size of the holes 53 in mesh plate 52 and the surface tension of the liquid. The drops expelled from plate 52 pass through tubular projection 16 of housing 22 into the patient limb 10 and are inhaled by the subject as nebulized liquid.

As noted above, valve 18, which is used for controlling the liquid supply from reservoir 60 to vibrating mesh plate 52, is formed of a fixed part 39 and a changeable or disposable part 69. The fixed part 39 is located inside plug member 30 and the disposable part 69 is affixed to liquid reservoir 60. The disposable part 69 includes elements that regulate the liquid flow through the valve under the influence of a magnetic field, which is produced with the fixed part 39. As noted above, the disposable part 69 by itself functions as a shut off, preventing liquid flow from reservoir 60. Disposable part 69, together with fixed part 39, functions as an electrically controlled flow valve. Disposable part 69 and fixed part 39 of the valve are shown in more detail in Fig. 5a-1 and Fig. 5a-2.

Disposable part 69 comprises a disk-like plate 70, which is attached to, or incorporated in, plate 64 of reservoir 60 (not shown in Fig. 5a). Plate 70 has opening 71. Liquid conveying tube 73 depends from plate 70. A stopper 75, in which may have a shape resembling a bullet, is placed in tube 73. Spring 76 may also be placed in tube 73. Plate 70 and stopper 75 are made of ferromagnetic material such as stainless steel. The upper end of tube 73 is connected to a tubular connector 77 of plate 70. The lower end of tube 73 forms an opening 74. Spring 76, if used, is interposed between plate 70 and stopper 75 so that the spring 76 biases the stopper 75 against the valve seat 80 formed at edges of opening 74. The pressure of the liquid in reservoir 60 may also be used to press the stopper against the valve seat.

Fig. 5b shows details of two examples of stopper 75. Stopper 75 shown in Figs. 5b-1 and 5b-2 has lengthwise channels 78 on the cylindrical side wall, which are used to direct the fluid flow past the stopper from opening 71 to opening 74. There is u circular step 79 at the upper end of stopper 75, resembling a corresponding step at the lower end of tubular connection 77 of plate 70, to accommodate spring 76, if used. The upper surface of stopper 75 and lower surface of tubular connection 77 of plate 70 may abut when stopper 75 is raised.

Figs. 5b-3 and 5b-4 show another embodiment of stopper 75 having a generally rectangular cross-sectional configuration. Stopper 75 shown in Figs. 5b-3 and 5b-4 can be easily manufactured by cutting off a piece from a generally rectangular bar having truncated corners. The spaces 78 that are present along the sides of the stopper function as the channels to allow fluid flow past the stopper when the valve is open.

As shown in Fig. 5a-2, fixed part 39 comprises a solenoid or a coil 41 formed of an insulated electric wire, such as copper having an enameled coating. Both ends of the wire are brought through the cylindrical, ferromagnetic body 42 covering the coil 41 and connected to conductor 100 and cable 20 via appropriate power supply conductors 43. Coil 41 can be wound around, for example, a thin plastic reel (not shown in Fig. 5a-2) or it can be cast in resin so that there is an opening through the coil and the coil is then fitted inside the body 42. The sensing electrode 38, which is also made of ferromagnetic material, is formed at an opening at the lower end of cylindrical body 42 and it is also connected to conductor 100 and cable 20, as by conductor 45, to enable the impedance measurement used for detecting the liquid supplied to the mesh plate.

Disposable part 69 is formed so that it fits into, and extends through, the tubular cavity 40 of the fixed part 39. When the reservoir 60 is attached to plug member 30, tubular disposable part 69 extends through cavity 40 so that the lower end of tube 73 is approximately at the height of lower end of sensing electrode 38.

The functioning of the disposable part 69, when attached to fixed part 39, is shown in more detail in Fig. 6. The inactivated condition of valve 18 is shown in Fig. 6a. Stopper 75 is forced against valve seat 80 in tube 73 preventing the flow liquid from reservoir 60 through tube 73 to the upper surface of mesh plate 52. When the coil 41 is connected to a voltage source, the current produces a magnetic field, as shown Fig. 6b. Due to the resulting solenoid action, the valve tries to reach an equilibrium state by moving stopper 75 upwardly toward plate 70 of reservoir 60 as shown in Fig. 6b. When stopper 75 moves toward plate 70, a channel is opened through valve seat 80 from opening 71 in plate 70 to opening 74 in tube 73 and the liquid flows through the tube 73 past the stopper 75 along channels 78 onto mesh plate 52.

When reservoir 60 is attached to plug member 30, ferromagnetic metal plate 70 should firmly abut ferromagnetic body 42 to provide the best possible magnetic circuit for the flux of the magnetic field of coil 41. Stopper 75, which is also made of ferromagnetic material, or a combination of ferromagnetic material and another material such as rubber, moves freely responsive to the magnetic field, except for the pressure of spring 76 and/or the fluid in reservoir 60, inside the tube 73. A portion of the magnetic field circuit extends between sensing electrode 38 and stopper 75 and the tube 73 and the field strength weakens less, the shorter the distance between stopper 75 and sensing electrode 38. That means the thinner the wall of tube 73, the better the magnetic circuit. The same concerns govern the spacing between stopper 75 and plate 70. The lower magnetic field losses in the structure, the lower the amplitude of voltage that is required for controlling the valve.

Figs. 7 and 8 show another embodiment of a valve suitable for use in a liquid discharge apparatus. In this embodiment, a valve is provided internally of liquid reservoir 60. To this end, valve disk 100 is placed over opening 102 in plate 64 of reservoir 60. Means are provided to bias valve disk 100 into contact with plate 64 to close opening 102. Such means may comprise a spring in a cage mounted on plate 64 or elastic threads 104 fastened to plate 64 and arranged in a cross over valve disk 100. The pressure of the liquid when reservoir 60 is filled also serves to bias the disk into contact with plate 64 and close opening 102.

The valve of Figs. 7 and 8 also includes intermediate part 108 formed of tube 110. Actuator member 112 is loosely placed in tube 110 for movement from a lower position in which member 112 rests against shoulder 114 and a raised position. Actuator member 112 may generally be formed in the same or similar manner as stopper 75 shown in Fig. 5b. The upper end of tube 110 is closed by a plate 116 of ferromagnetic material containing hole 118. Projection 120 of actuator member 112 extends through hole 118. Tube 110 has opening 122 in the lower portion.

The fixed part 39 of the valve shown in Figs. 7 and 8 generally resembles that shown in Figs. 5a and 6 as indicated by the common reference numerals.

In the embodiment of the invention shown in Figs. 7 and 8, intermediate part 108 may be attached to reservoir 60 in the manner shown in Fig. 2. Or, intermediate part 108 may be affixed inside fixed portion 39 of the valve. Or, intermediate part 108 may be a component separate from either reservoir 60 or fixed part 39. If intermediate part 108 is fastened to reservoir 60, it can be discarded with the reservoir at the end of treatment. If it is a separate component, it can be discarded or cleaned for rcuse. If it is fastened to fixed part 39, it can be cleaned after use, along with the other components of liquid discharge apparatus 1.

In use, intermediate part 108 is placed in fixed part 39, either separately, or, if intermediate part 108 is fastened to reservoir 60, when reservoir 60 is attached to plug member 30. When so assembled, the upper end of projection 120 is proximate to, but spaced from, valve disk 100 in reservoir 60. When coil 124 is energized, actuator member 112 moves from the lower position shown in Fig. 8a to the raised position shown in Fig. 8b. Projection 120 extending through holes 118 and 102 lifts valve disk 100 off plate 64, allowing liquid in reservoir 60 to flow out opening 102 through tube 110 and into apparatus 1. Shoulder 114 may be formed as a valve seat for member 108, if desired, to provide an additional means for preventing liquid flow from reservoir 60 to nebulizer apparatus 1 when member 108 is in the lower position.

Figs. 9 and 10 show a further embodiment of a valve suitable for use in a liquid discharge apparatus. In this embodiment, and similar to the embodiment of Figs. 7 and 8, a valve is provided internally of liquid reservoir 60. Valve disk 100 is placed over opening 102 in plate 64 of reservoir 60 and biased into contact with plate 64 as in one of the manners described in connection with Figs. 7 and 8. Tube 210 extends from opening 102 in plate 64.

An elastic area is provided in plate 64. The elastic area 213 is defined by ring shaped channel 211 having a smaller thickness than plate 64. The elastic area 213 can be formed in plate 64 by multi component fabrication techniques using, for example, plastics and rubber.

An actuator member 212 is placed in magnetic coil 124. Actuator member 212 may comprise a ferromagnetic sleeve which is loosely placed in a tubular recess in magnetic coil 124. The length of actuator 212 is less than the length of the recess so that actuator member may move upward when the magnetic coil is energized from a lower position in which the actuator rests against shoulder 214. Projection 220 is mounted on the upper end of actuator member 212 and extends through hole 218 in the ferromagnetic body 123 of magnetic coil 124.

When liquid reservoir 60 is attached to plug member 30 the upper end of projection 220 is proximate to, but spaced from, elastic area 213. Tube 210 extends through actuator member 212. When coil 124 is energized, actuator member 212 moves from lower position shown in Fig. 10a to the raised position shown in Fig. 10b. Projection 220 extends through hole 218 and presses on elastic area 213. Elastic area 213 moves upwardly and lifts valve disk 100 off plate 64, allowing liquid in reservoir 60 to flow out opening 102 through tube 210 into apparatus 1.

It is recognized that other equivalents, alternatives, and modifications aside from those expressly stated, are possible and within the scope of the appended claims.

## Claims

1. An apparatus for discharging liquid comprising:
a housing having an opening through which the liquid is discharged;
a displaceable element in said housing which is subject to displacement for discharging liquid;
means for displacing said element to cause liquid to discharge from said opening;
a liquid reservoir;
liquid conveying means extending from said reservoir into proximity with said displaceable element;
means for controlling the flow of liquid through said liquid conveying means, said liquid flow controlling means being coupled to said reservoir;
a magnetic field source for selectively producing a magnetic field, and
a ferromagnetic member positioned to lie in the magnetic field, when formed, said ferromagnetic field coacting with said liquid flow controlling means and being movable responsive to the production of the magnetic field to allow liquid to flow through said liquid flow controlling means from said reservoir to said displaceable clement to be discharged from said apparatus.

2. An apparatus according to claim 1 wherein said liquid flow controlling means comprises a valve seat in said liquid conveying means for coacting with a ferromagnetic member comprising a stopper, said ferromagnetic stopper being in said liquid conveying means and moved to open said valve seat responsive to the production of the magnetic field to allow liquid to flow through said valve seat.

3. An apparatus according to claim 1 wherein said liquid flow controlling means comprises a valve mounted on said reservoir and wherein said ferromagnetic member comprises an actuator, said actuator being moved to open said valve responsive to the production of the magnetic field to allow liquid to flow through said valve.

4. An apparatus according to claim 3 wherein said actuator is located in said liquid conveying means.

5. An apparatus according to claim 3 wherein said actuator is located externally of said liquid conveying means.

6. An apparatus according to claim 2 or 3 wherein said liquid conveying means is mounted on said reservoir.

7. An apparatus according to claim 3 wherein said liquid conveying means is an element separate from said reservoir and housing.

8. An apparatus according to claim 1 or 2 wherein said ferromagnetic member has peripheral channels for allowing liquid flow through said liquid conveying means.

9. An apparatus according to claim 1 wherein said ferromagnetic member is circular in cross section.

10. An apparatus according to claim 2 further including bias means to bias said stopper toward said valve seat.

11. An apparatus according to claim 1 wherein said liquid conveying means is a thin walled tube.

12. An apparatus according to claim 1 wherein said magnetic field source comprises a coil and wherein said liquid conveying means extends through said coil.

13. An apparatus according to claim 1 wherein said displaceable element is conductive and wherein said apparatus has further conductive means extending toward said displaceable element for measuring, in conjunction with said displaceable element, an impedance indicative of the amount of liquid provided to said displaceable element.

14. An apparatus according to claim 1 wherein said liquid reservoir is removably attachable to said apparatus.

15. An apparatus according to claim 1 wherein said reservoir is formed to pressurize the liquid therein.

16. An apparatus according to claim 1 wherein said displaceable element has at least one aperture through which liquid passes to said opening.

17. An apparatus according to claim 1 wherein said displacing means comprises piezoelectric means.

18. An apparatus according to claim 1 wherein said displaceable element is further defined as a vibratable element.

19. An apparatus according to claim 1 wherein said displaceable element is subject to bowing to create the displacement.

20. An apparatus according to claim 18 wherein said vibratable element includes a mesh plate for atomizing the liquid.

21. An apparatus according to claim 13 further including element coupled to said apparatus for energizing said magnetic field source responsive to the impedance measurement.

22. An apparatus according to claim 1, 2, 4 or 13 wherein said apparatus is further defined as a nebulizer for discharging atomized liquid.

## Patentansprüche

1. Vorrichtung zum Ausstoßen von Flüssigkeit, umfassend:
- ein Gehäuse mit einer Öffnung, durch welche die Flüssigkeit ausgestoßen wird;
- ein bewegbares Element in dem Gehäuse, welches zum Ausstoß der Flüssigkeit bewegt wird;
- Mittel um dieses Element zu bewegen, um Flüssigkeit dazu zu bringen von dieser Öffnung abzufließen;
- ein Flüssigkeitsreservoir;
- Flüssigkeitstransportmittel, welche sich von dem Reservoir in die Nähe des bewegbaren Elements erstrecken;
- Mittel um den Flüssigkeitsfluss durch das Flüssigkeitstransportmittel zu steuern, welche Flüssigkeitsfluss-Steuerungsmittel an dem Reservoir angekoppelt sind;
- eine Magnetfeldquelle um selektiv ein Magnetfeld zu produzieren, und
- ein ferromagnetisches Bauteil, welche positioniert ist, um in dem Feld zu liegen, wenn dieses gebildet ist, welches ferromagnetische Feld mit dem Flüssigkeitsfluss-Steuerungsmittel zusammenwirkt und reagierend auf die Erzeugung des Magnetfelds bewegbar ist, um den Fluss von Flüssigkeit durch das Flüssigkeitsfluss-Steuerungsmittel von dem Reservoir zu dem bewegbaren Element zu erlauben, um von der Vorrichtung ausgestoßen zu werden.

2. Vorrichtung nach Anspruch 1,
**dadurch gekennzeichnet, dass** das Flüssigkeitsfluss-Steuerungsmittel einen Ventilsitz in dem Flüssigkeitstransportmittel umfasst, um mit einem ferromagnetischen Bauteil zusammen zu wirken, welches einen Stopper umfasst, welcher ferromagnetische Stopper in dem Flüssigkeitstransportmittel ist und bewegt wird, um reagierend auf die Erzeugung des Magnetfelds den Ventilsitz zu öffnen, um den Fluss von Flüssigkeit durch den Ventilsitz zu erlauben.

3. Vorrichtung nach Anspruch 1,
**dadurch gekennzeichnet, dass** das Flüssigkeitsfluss-Steuerungsmittel ein Ventil umfasst, welches an dem Reservoir montiert ist und wobei das ferromagnetische Bauteil ein Betätigungselement umfasst, welches Betätigungselement bewegt wird, um das Ventil reagierend auf die Erzeugung des Magnetfelds zu öffnen, um den Fluss von Flüssigkeit durch das Ventil zu erlauben.

4. Vorrichtung nach Anspruch 3,
**dadurch gekennzeichnet, dass** das Betätigungselement in dem Flüssigkeitstransportmittel angeordnet ist.

5. Vorrichtung nach Anspruch 3,
**dadurch gekennzeichnet, dass** das Betätigungselement außerhalb von dem Flüssigkeitstransportmittel angeordnet ist.

6. Vorrichtung nach Anspruch 2 oder 3,
**dadurch gekennzeichnet, dass** das Flüssigkeitstransportmittel an dem Reservoir montiert ist.

7. Vorrichtung nach Anspruch 3,
**dadurch gekennzeichnet, dass** das Flüssigkeitstransportmittel ein von dem Reservoir und Gehäuse separates Element ist.

8. Vorrichtung nach Anspruch 1 oder 2,
**dadurch gekennzeichnet, dass** ferromagnetische Bauteil periphere Kanäle hat, um einen Flüssigkeitsfluss durch das Flüssigkeitstransportmittel zu erlauben.

9. Vorrichtung nach Anspruch 1,
**dadurch gekennzeichnet, dass** das ferromagnetische Bauteil im Querschnitt kreisförmig ist.

10. Vorrichtung nach Anspruch 2,
welche weiter Wirkmittel enthält, um den Stopper gegen den Ventilsitz zu drängen.

11. Vorrichtung nach Anspruch 1,
**dadurch gekennzeichnet, dass** das Flüssigkeitstransportmittel ein dünnwandiges Rohr ist.

12. Vorrichtung nach Anspruch 1,
**dadurch gekennzeichnet, dass** die Magnetfeldquelle eine Spule umfasst und worin sich das Flüssigkeitstransportmittel durch diese Spule erstreckt.

13. Vorrichtung nach Anspruch 1,
**dadurch gekennzeichnet, dass** das bewegbare Element leitfähig ist and worin die Vorrichtung weitere leitfähige Mittel hat, welche sich zu dem bewegbaren Element erstrecken, um, in Verbindung mit dem bewegbaren Element, eine Impedanz zu messen, welche auf die Menge an zu dem bewegbaren Element zugeführter Flüssigkeit hinweist.

14. Vorrichtung nach Anspruch 1,
**dadurch gekennzeichnet, dass** das Flüssigkeitsreservoir entfernbar an der Vorrichtung befestigbar ist.

15. Vorrichtung nach Anspruch 1,
**dadurch gekennzeichnet, dass** das Flüssigkeitsreservoir geformt ist, um die Flüssigkeit darin unter Druck zu setzen.

16. Vorrichtung nach Anspruch 1,
**dadurch gekennzeichnet, dass** das bewegbare Element wenigstens einen Durchlass hat, durch welchen Flüssigkeit zu der Öffnung fließt.

17. Vorrichtung nach Anspruch 1,
**dadurch gekennzeichnet, dass** das bewegbare Element piezoelektrische Elemente umfasst.

18. Vorrichtung nach Anspruch 1,
**dadurch gekennzeichnet, dass** das bewegbare Element ein vibrierfähiges Element ist.

19. Vorrichtung nach Anspruch 1,
**dadurch gekennzeichnet, dass** das bewegbare Element einer Biegung ausgesetzt ist, um die Bewegung zu erzeugen.

20. Vorrichtung nach Anspruch 18,
**dadurch gekennzeichnet, dass** das vibrierfähige Element eine Netzplatte zur Zerstäubung der Flüssigkeit enthält.

21. Vorrichtung nach Anspruch 13,
welche weiter ein an der Vorrichtung angekoppeltes Element enthält, um die Magnetfeldquelle reagierend auf die Impedanz Messung mit Strom zu versorgen.

22. Vorrichtung nach Anspruch 1, 2, 4 oder 13,
**dadurch gekennzeichnet, dass** die Vorrichtung ein Zerstäuber zum Ausstoß von zerstäubter Flüssigkeit ist.

## Revendications

1. Appareil pour projeter un liquide comprenant :
un boîtier muni d'un orifice à travers lequel le liquide est projeté ;
un élément déplaçable dans ledit boîtier qui est soumis à un déplacement pour projeter le liquide ;
un moyen pour déplacer ledit élément afin de provoquer la projection du liquide depuis ledit orifice ;
un réservoir de liquide ;
un moyen d'acheminement du liquide qui s'étend dudit réservoir à un endroit à proximité dudit élément déplaçable ;
un moyen de contrôle de l'écoulement de liquide dans ledit moyen d'acheminement du liquide, ledit moyen de contrôle de l'écoulement de liquide étant couplé audit réservoir ;
une source de champ magnétique pour produire d'une manière sélective un champ magnétique, et
un organe ferromagnétique positionné de façon à se trouver dans le champ magnétique, quand formé, ledit champ ferromagnétique agissant en même temps que ledit moyen de contrôle de l'écoulement de liquide et étant mobile en réponse à la production du champ magnétique pour permettre au liquide de s'écouler à travers ledit moyen de contrôle de l'écoulement de liquide depuis ledit réservoir jusqu'audit élément déplaçable afin d'être projeté depuis ledit appareil.

2. Appareil selon la revendication 1 dans lequel ledit moyen de contrôle de l'écoulement de liquide comprend un siège de soupape dans ledit moyen d'acheminement du liquide pour agir en même temps qu'un organe ferromagnétique comprenant un bouchon, ledit bouchon ferromagnétique étant situé dans ledit moyen d'acheminement du liquide et déplacé pour ouvrir ledit siège de soupape en réponse à la production du champ magnétique afin de permettre au liquide de s'écouler à travers ledit siège de soupape.

3. Appareil selon la revendication 1 dans lequel ledit moyen de contrôle de l'écoulement de liquide comprend une soupape montée sur ledit réservoir et dans lequel ledit organe ferromagnétique comprend un actionneur, ledit actionneur étant déplacé pour ouvrir ladite soupape en réponse à la production du champ magnétique pour permettre au liquide de s'écouler à travers ladite soupape.

4. Appareil selon la revendication 3 dans lequel ledit actionneur est situé dans ledit moyen d'acheminement du liquide.

5. Appareil selon la revendication 3 dans lequel ledit actionneur est situé à l'extérieur dudit moyen d'acheminement du liquide.

6. Appareil selon la revendication 2 ou 3 dans lequel ledit moyen d'acheminement du liquide est monté sur ledit réservoir.

7. Appareil selon la revendication 3 dans lequel ledit moyen d'acheminement du liquide est un élément séparé dudit réservoir et boîtier.

8. Appareil selon la revendication 1 ou 2 dans lequel ledit organe ferromagnétique a des canaux périphériques pour permettre au liquide de s'écouler dans ledit moyen d'acheminement du liquide.

9. Appareil selon la revendication 1 dans lequel ledit organe ferromagnétique est circulaire en coupe transversale.

10. Appareil selon la revendication 2 qui comprend en outre un moyen de poussée pour pousser ledit bouchon vers ledit siège de soupape.

11. Appareil selon la revendication 1 dans lequel ledit moyen d'acheminement du liquide est un tube à paroi mince.

12. Appareil selon la revendication 1 dans lequel ladite source de champ magnétique comprend une bobine et dans lequel ledit moyen d'acheminement du liquide s'étend à travers ladite bobine.

13. Appareil selon la revendication 1 dans lequel ledit élément déplaçable est conducteur et dans lequel ledit appareil a d'autres moyens conducteurs s'étendant vers ledit élément déplaçable pour mesurer, en association avec ledit élément déplaçable, une impédance indiquant la quantité de liquide fournie audit élément déplaçable.

14. Appareil selon la revendication 1 dans lequel ledit réservoir de liquide peut être attaché d'une manière amovible audit appareil.

15. Appareil selon la revendication 1 dans lequel ledit réservoir est formé de façon à pressuriser le liquide à l'intérieur de celui-ci.

16. Appareil selon la revendication 1 dans lequel ledit élément déplaçable a au moins une ouverture à travers laquelle le liquide passe vers ledit orifice.

17. Appareil selon la revendication 1 dans lequel ledit élément déplaçable comprend un moyen piézoélectrique.

18. Appareil selon la revendication 1 dans lequel ledit élément déplaçable est en outre défini comme étant un moyen que l'on peut faire vibrer.

19. Appareil selon la revendication 1 dans lequel ledit élément déplaçable est soumis à une flexion pour créer le déplacement.

20. Appareil selon la revendication 18 dans lequel ledit élément que l'on peut faire vibrer incorpore une plaque perforée pour atomiser le liquide.

21. Appareil selon la revendication 13 incorporant en outre un élément couplé audit appareil pour exciter ladite source de champ magnétique en réponse à la mesure d'impédance.

22. Appareil selon la revendication 1, 2, 4 ou 13 dans lequel ledit appareil est en outre défini comme étant un nébuliseur pour projeter un liquide atomisé.
